(19) Europäisches Patentamt
European Patent Office
Office européen des brevets

(11) Veröffentlichungsnummer: 0 121 228
A2

(12) # EUROPÄISCHE PATENTANMELDUNG

(21) Anmeldenummer: 84103400.2

(22) Anmeldetag: 28.03.84

(51) Int. Cl.³: **C 12 P 19/44**
C 12 P 9/00, C 12 P 1/00
A 23 L 1/34, A 61 K 7/08
A 61 K 47/00, A 01 N 25/30
C 11 B 1/10

(30) Priorität: 02.04.83 DE 3312166

(43) Veröffentlichungstag der Anmeldung:
10.10.84 Patentblatt 84/41

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Kachholz, Traudel, Dr.
Schreyestrasse 6
D-6242 Kronberg/Taunus(DE)

(72) Erfinder: Schlingmann, Merten, Dr.
Schneidhainer Strasse 32a
D-6240 Königstein/Taunus(DE)

(54) **Lipotenside, Verfahren zu ihrer Isolierung und ihre Verwendung.**

(57) Durch Extraktion mikrobieller Zellmassen mit Lipidlösemitteln und Entfernung der Phospholipide aus diesen
Extrakten erhält man stark grenzflächenaktive Produkte. Diese
als Lipotenside bezeichneten Produkte eignen sich als grenzflächenaktive Mittel, insbesondere auf dem Lebensmittelsektor, für Pflanzenschutzmittel, Arzneimittel und Kosmetika.

EP 0 121 228 A2

Croydon Printing Company Ltd

HOECHST AKTIENGESELLSCHAFT     HOE 83/F 051   Dr **0121228**

Lipotenside, Verfahren zur ihrer Isolierung und ihre
Verwendung

Mikroorganismen enthalten wie alle Zellen neben Proteinen,
Kohlenhydraten und Nukleinsäuren auch Lipide. Bei der Aufarbeitung mikrobieller Zellmassen zur Gewinnung von Proteinen für die menschliche Ernährung werden außer den
Nukleinsäuren auch die Lipide abgetrennt, da diese den
Geruch, Geschmack und die Haltbarkeit der Proteine beeinträchtigen. Die Entfernung der Lipide erfolgt durch Extraktionen mit Lipidlösemitteln. Entsprechende Verfahren
sind beispielsweise in der DE-PS 26 33 666 genannt. Besonders bevorzugt ist das in dieser DE-PS beanspruchte
Verfahren, bei dem die Lipide mit einer Extraktionsmischung
aus Ammoniak und einem polaren Lösemittel aus der Reihe der
niederen Alkanole, niederen Glycole und der Methyl- oder
Ethylether eines niederen Glykols extrahiert werden, wobei
diese Extraktionsmischung höchstens 30 Gew.-% Wasser,
bezogen auf die eingesetzte Lösemittelmenge, enthält. Das
bevorzugte Lösemittel ist Methanol. Der Ammoniakgehalt
liegt vorzugsweise bei 1 bis 10 Gew.-%, bezogen auf die
eingesetzte Lösemittelmenge.

Aus der DE-OS 23 46 088 ist es bekannt, aus einem wäßrigen
Hefeisolat mit einem Lipidgehalt von 18,5 Gew.-% die Lipide
durch Extraktion mit einem Gemisch aus 80 % Hexan und 20 %
Ethanol zu isolieren. Der so erhaltene Lipidextrakt dient
zur Herstellung eines Lederfettungsmittels. Die Verwendung
eines Lipidextrakts aus Methanol verwertenden Bakterien zum
Fetten von Leder und Pelzfellen ist außerdem aus der europäischen Patentanmeldung 15 439 bekannt. Der dort verwendete
Lipidextrakt besteht zu über 60 % aus Phospholipiden und
enthält außerdem noch triglyceridische Fette und freie
$C_{16}$-Fettsäuren.

Phospholipide sind auf Grund ihres Gehaltes an polaren und
unpolaren Gruppen grenzflächenaktiv und finden deshalb als
Tenside Verwendung, beispielsweise in der Lebensmitteltechnologie. Überraschenderweise wurde jedoch gefunden, daß die Lipidextrakte aus mikrobiellen Zellmassen sehr viel stärker oberflächenaktiv wirken als es deren Gehalt an Phospholipiden und freien Fettsäuren erwarten läßt. Der Lipidextrakt muß somit noch hochwirksame grenzflächenaktive Komponenten enthalten, die hier als "Lipotenside" bezeichnet werden. Die Erfindung betrifft ein Verfahren zur Isolierung dieser Lipotenside, das dadurch gekennzeichnet ist, daß man mikrobielle Zellmassen mit Lipidlösemitteln extrahiert und aus dem Extrakt die Phospholipide aufgrund ihrer geringeren Löslichkeit abtrennt, z. B. ausfällt.

Als Ausgangsmaterial kommen grundsätzlich alle mikrobiellen Zellmassen in Betracht, da - wie eingangs erwähnt - alle Zellen Lipide enthalten. Bevorzugt sind methylotrophe Organismen, insbesondere Methanol verwertende Mikroorganismen, da Methanol eine billige, Erdöl-unabhängige Kohlenstoffquelle darstellt. Obligat methylotrophe Organismen sind wegen ihrer "biologischen Sicherheit" bevorzugt, aber auch deshalb, weil aus ihnen hochwirksame Lipotenside isoliert werden können. Methanol verwertende Mikroorganismen sind in großer Zahl bekannt und beispielsweise in den europäischen Patentanmeldungen 35 831 und 37 273, in der DE-AS 21 61 164 und insbesondere in der DE-PS 26 33 451 beschrieben.

Es ist vorteilhaft, wenn die mikrobiellen Zellmassen vor der Extraktion der Lipide durch eine Hitzebehandlung bei Temperaturen von 105 bis 160°C konditioniert werden. Diese Hitzebehandlung dauert vorteilhaft 3 bis 40, insbesondere 5 bis 20, Minuten bei einer Produkt-Temperatur von 105 bis 140°C. Ein solches Verfahren ist Gegenstand der Deutschen Patentanmeldung P 33 08 024.0; es führt nicht nur zu einer Verbesserung der mechanischen Eigenschaften der mikrobiellen Rohbiomasse, die einen geringeren Proteinverlust in der folgenden Extraktion ergibt, sondern hat auch einen vorteilhaften Einfluß auf die physiologischen Eigenschaften der Proteine.

Wie bereits vorstehend erwähnt, ist als Extraktionsverfahren für die Lipide die Extraktion gemäß DE-PS 26 33 666 bevorzugt. Dieses Verfahren ist nicht nur hinsichtlich seiner Extraktionsleistung besonders vorteilhaft, sondern es erfordert auch wenig Energie und ist außerordentlich schonend für das Protein.

Zur Isolierung der Lipotenside können die Lipidextrakte zur Trockene eingedampft werden. Im Falle der gemäß DE-PS 26 33 666 erhaltenen ammoniakhaltigen Extrakte reicht es jedoch aus, diese mehr oder weniger einzuengen, wobei das Ammoniak zurückgewonnen wird. Aus der konzentrierten alkoholischen Lösung können dann die Phospholipide durch Zusätze ausgefällt werden, die die Löslichkeit der Phospholipide verringern. Solche Zusätze sind mit dem Alkohol mischbare Lösemittel, die jedoch für die Phospholipide keine guten Lösemittel darstellen, insbesondere niedere Alkylester niedermolekularer aliphatischer Carbonsäuren wie Essigsäuremethyl- oder -ethylester oder niedere aliphatische Ketone wie Aceton oder Methylethyl-keton, oder aber Salze mehrwertiger Metallionen, beispielsweise zweiwertiger Metallionen wie Calciumchlorid.

Wenn das für die Extraktion der Lipide verwendete Lösemittel bzw. Lösemittelgemisch eine solche selektive Fällung nicht erlaubt, so kann der Extrakt zur Trockene eingedampft werden und der Rückstand in einem niederen Alkohol, vorzugsweise Methanol, aufgenommen werden, worauf die Ausfällung der Phospholipide wie vorstehend angegeben erfolgt. Möglich ist auch eine Extraktion der Lipotenside, bei der die Phospholipide ungelöst zurückbleiben. Hierfür kommt beispielsweise ein Methanol-Aceton-Gemisch in Betracht.

Die auf diese Weise erhaltenen Phospholipid-Rückstände können in üblicher Weise aufgearbeitet werden. Aus den Filtraten bzw. Überständen können die Lipotenside durch Eindampfen gewonnen werden.

Engt man beispielsweise einen methanolisch-ammoniakalischen Lipidextrakt, der nach Beispiel 1 der DE-PS 26 33 666 erhalten wurde und 20 g Fett pro Liter enthält, auf etwa 1/10 seines Volumens ein und gibt dann etwa die 4-fache Menge, bezogen auf das eingeengte Volumen des Extrakts, an Aceton zu, so fallen etwa 60 Gew.-% der im Extrakt enthaltenen Feststoffe aus. Dieser Niederschlag besteht im wesentlichen aus Phospholipiden und kann in üblicher Weise gereinigt werden.

Engt man den methanolisch-ammoniakalischen Extrakt auf etwa 1/5 seines Volumens ein und setzt dann pro Liter etwa 0,1 bis 3 g Calciumchlorid-Heptahydrat zu, so erhält man ebenfalls eine Fällung von etwa 60 Gew.-% der im Extrakt vorhandenen Feststoffe. Auch dieser Niederschlag kann in bekannter Weise auf Phospholipide aufgearbeitet werden.

Beim Eindampfen der Filtrate bzw. Überstände wird jeweils ein Rückstand erhalten, der etwa 40 Gew.-% der ursprünglich im Extrakt enthaltenen Feststoffe darstellt. Dieser Rückstand besteht aus den Lipotensiden.

Zur Isolierung einer besonders wirksamen grenzflächenaktiven Komponente kann dieser Rückstand in einem unpolaren Lösemittel, zweckmäßig in einem niedrig siedenden Kohlenwasserstoff wie Pentan, Hexan, Heptan, Cyclohexan oder einem Kohlenwasserstoffgemische wie Petrolether, in der Wärme gelöst werden, worauf beim Abkühlen ein Niederschlag auskristallisiert bzw. ausfällt. Es ist auch möglich, die Rückstände mit solchen unpolaren Lösemitteln zu extrahieren. Aus diesen Lösungen in dem unpolaren Lösemittel wird dann die hochwirksame Komponente durch Eindampfen isoliert. Man erhält etwa 30 Gew.-% des Feststoffgehalts im urprünglichen Lipidextrakt.

Aus dem Lipidextrakt gemäß Beispiel 1 der DE-PS 26 33 666 wird so ein Produkt erhalten, das als Glycolipid charakterisiert werden konnte. Dessen Kohlenhydratanteil besteht im wesentlichen aus Glucose und Rhamnose und dessen Lipidanteil im wesentlichen aus Palmitinsäure und Palmitoleinsäure. Dieses Glycolipid ist stark grenzflächenaktiv.

Auf Grund der Grenzflächenaktivität eignen sich die Lipotenside als hochwertige Tenside. Als Naturprodukte sind sie leicht biologisch abbaubar und von geringer Toxizität und eignen sich deshalb besonders für spezielle Einsatzzwecke wie im Lebensmittelsektor, für Pflanzenschutzmittel, Arzneimittel oder Kosmetika.

Für den Einsatz als Tensid ist es in vielen Fällen nicht erforderlich, die Begleitsubstanzen der Lipotenside abzutrennen. So stören für die meisten Einsatzzwecke die freien Fettsäuren und oft auch ein mehr oder weniger hoher Anteil der Phospholipide nicht. Je nach Einsatzzweck wird der Fachmann hierbei den mit der Abtrennung verbundenen Aufwand in Relation zur verbesserten Tensidwirkung setzen und so die zweckmäßigste Anreicherungsstufe wählen.

Für die folgenden Beispiele wurde eine Bakterienmasse aus Methylomonas clara ATCC 31 226 gemäß DE-PS 26 33 451, Beispiel 2, mit einem Rohfettgehalt von 5 bis 10 % und einer Restfeuchte von 2 bis 4 % eingesetzt. Dieses Material wurde 30 Minuten in einem Wirbelbett bei 160°C Lufttemperatur behandelt, wobei 10 Minuten eine Produkttemperatur von 120°C eingehalten wurde. Diese thermisch nachbehandelte Zellmasse wurde dann gemäß Beispiel 1 der DE-PS 26 33 666 mit methanolischem Ammoniak extrahiert. Der Extrakt enthielt 20 g Fett pro Liter. Prozentangaben beziehen sich hier und im folgenden auf das Gewicht.

Beispiel 1

500 ml Extrakt werden auf 50 ml eingeengt, wobei das Ammoniak mit dem übergehenden Methanol praktisch vollständig entfernt wird. Das Destillat wird in den Extraktionsprozeß zurückgeführt.

Zu dem eingeengten Extrakt werden 200 ml Aceton gegeben, wobei ein im wesentlichen aus Phospholipiden bestehender Niederschlag ausfällt. Der Überstand wird dekantiert und fraktioniert destilliert. Das wiedergewonnene Aceton wird bei einem folgenden Ansatz zur Fällung eingesetzt, das Methanol kann in die Extraktion zurückgeführt werden. Als Rückstand erhält man 4 g eines stark grenzflächenaktiven Produkts.

Beispiel 2

500 ml des Extrakts werden auf 100 ml eingeengt, wobei ebenfalls das Ammoniak weitestgehend mit dem Methanol entfernt wird. Dieses Destillat wird in die Extraktion zurückgeführt.

Zum eingeengten Rückstand gibt man 0,15 g Calciumchlorid-Heptahydrat. Man erhält 6,5 g eines Niederschlags, der im wesentlichen aus Phospholipiden besteht. Aus dem Überstand erhält man durch Einengen 4 g eines stark grenzflächenaktiven Produktes.

Beispiel 3

Das nach Beispiel 1 oder 2 erhaltene stark grenzflächenaktive Produkt wird in 40 ml Hexan in der Wärme gelöst (etwa 35°C). Beim Abkühlen dieser Lösung erhält man 1 g eines Kristallisats, das abfiltriert wird. Aus dem Filtrat erhält man nach Eindampfen 3 g Glycolipid, dessen Kohlenhydratanteil im wesentlichen aus Glucose und Rhamnose und dessen Lipidanteil im wesentlichen aus Palmitinsäure (42 %) und Palmitoleinsäure (50 %) bestehen.

Beispiel 4

Die Oberflächenspannung wäßriger Lösungen der Produkte aus den vorhergehenden Beispielen wurde mit einem Digital-Tensiometer nach der Platinringmethode (Ringabreißmethode, DIN 53 914) wie folgt bestimmt:

Oberflächenspannung in $10^{-3}$N/m

| Konzentration, % | Beispiel 1 | Beispiel 2 | Beispiel 3 |
|---|---|---|---|
| 1 | 34,2 | 31,1 | 29,9 |
| 0,1 | 34,8 | 33,6 | 30,2 |
| 0,01 | 34,9 | 34,0 | 30,1 |
| 0,005 | 35,3 | 34,5 | 32,3 |
| 0,001 | 42,4 | 34,7 | 33,1 |
| 0,0005 | 63,6 | 42,4 | 40,5 |
| 0,0001 | 65,7 | 63,5 | 50,7 |

Patentansprüche:

1. Verfahren zur Isolierung von Lipotensiden, dadurch gekennzeichnet, daß man mikrobielle Zellmassen mit Lipidlösemitteln extrahiert und aus dem Extrakt die Phospholipide aufgrund ihrer geringeren Löslichkeit abtrennt.

2. Verfahren nach Anspruch 1, dadurch gekennzeichnet, daß als mikrobielle Zellmassen solche von methylotrophen, vorzugsweise von Methanol verwertenden, Organismen, insbesondere von Methylomonas clara ATCC 31 226, eingesetzt werden.

3. Verfahren nach Anspruch 1 oder 2, dadurch gekennzeichnet, daß man vor der Extraktion die mikrobiellen Zellmassen durch eine Hitzebehandlung bei Temperaturen von 105 bis 160°C, vorzugsweise 3 bis 40 Minuten bei einer Produkt-Temperatur von 105 bis 140°C, konditioniert.

4. Verfahren nach einem oder mehreren der Ansprüche 1 bis 3, dadurch gekennzeichnet, daß man die Extraktion der Lipide mit einer Extraktionsmischung aus Ammoniak und einem polaren Lösemittel aus der Reihe der niederen Alkanole, niederen Glycole und der Methyl- oder Ethylether eines niederen Glycols vornimmt, wobei diese Extraktionsmischung höchstens 30 Gew.-% Wasser, bezogen auf die eingesetzte Lösemittelmenge, enthält.

5. Verfahren nach einem oder mehreren der Ansprüche 1 bis 4, dadurch gekennzeichnet, daß man zur Entfernung der Phospholipide den alkoholischen Extrakten einen niederen Alkylester einer niedermolekularen aliphatischen Carbonsäure, ein niedermolekulares aliphatisches Keton oder ein Salz eines mehrwertigen Metallions zusetzt.

6. Verfahren nach einem oder mehreren der Ansprüche 1 bis 5, dadurch gekennzeichnet, daß man nach Entfernung der Phospholipide die Extraktrückstände mit Hilfe eines unpolaren Lösemittels reinigt.

7. Verwendung der nach Anspruch 1 erhältlichen Lipotenside als grenzflächenaktive Mittel.

8. Verwendung der nach Anspruch 1 erhältlichen Lipotenside als grenzflächenaktive Mittel im Lebensmittelsektor, für Pflanzenschutzmittel, Pharmazeutica oder Kosmetika.

9. Lipotenside, erhältlich aus methylotrophen Organismen durch Extraktion mit Lipidlösemitteln und Entfernung der Phospholipide.

10. Lipotenside nach Anspruch 9 aus Methylomonas clara ATCC 31 226.

Patentansprüche für Österreich:

1. Verfahren zur Isolierung von Lipotensiden, dadurch gegekennzeichnet, daß man mikrobielle Zellmassen mit Lipidlösemitteln extrahiert und aus dem Extrakt die Phospholipide aufgrund ihrer geringeren Löslichkeit abtrennt.

2. Verfahren nach Anspruch 1, dadurch gekennzeichnet, daß als mikrobielle Zellmassen solche von methylotrophen, vorzugsweise von Methanol verwertenden, Organismen, insbesondere von Methylomonas clara ATCC 31 226, eingesetzt werden.

3. Verfahren nach Anspruch 1 oder 2, dadurch gekennzeichnet, daß man vor der Extraktion die mikrobiellen Zellmassen durch eine Hitzebehandlung bei Temperaturen von 105 bis 160°C, vorzugsweise 3 bis 40 Minuten bei einer Produkt-Temperatur von 105 bis 140°C, konditioniert.

4. Verfahren nach einem oder mehreren der Ansprüche 1 bis 3, dadurch gekennzeichnet, daß man die Extraktion der Lipide mit einer Extraktionsmischung aus Ammoniak und einem polaren Lösemittel aus der Reihe der niederen Alkanole, niederen Glycole und der Methyl- oder Ethylether eines niederen Glycols vornimmt, wobei diese Extraktionsmischung höchstens 30 Gew.-% Wasser, bezogen auf die eingesetzte Lösemittelmenge, enthält.

5. Verfahren nach einem oder mehreren der Ansprüche 1 bis 4, dadurch gekennzeichnet, daß man zur Entfernung der Phospholipide den alkoholischen Extrakten einen niederen Alkylester einer niedermolekularen aliphatischen Carbon-säure, ein niedermolekulares aliphatisches Keton oder ein Salz eines mehrwertigen Metallions zusetzt.

6. Verfahren nach einem oder mehreren der Ansprüche 1 bis 5, dadurch gekennzeichnet, daß man nach Entfernung der Phospholipide die Extraktrückstände mit Hilfe eines unpolaren Lösemittels reinigt.

7. Verwendung der nach Anspruch 1 erhältlichen Lipotenside als grenzflächenaktive Mittel.

8. Verwendung der nach Anspruch 1 erhältlichen Lipotenside als grenzflächenaktive Mittel im Lebensmittelsektor, für Pflanzenschutzmittel, Pharmazeutica oder Kosmetika.